# FASCICULE DE BREVET EUROPEEN

(11) **EP 1 126 829 B1**
(45) Date de publication et mention de la délivrance du brevet: **28.04.2004**
(21) Numéro de dépôt: 99954047.9
(22) Date de dépôt: 03.11.1999
(51) Int. Cl.: A61K 9/50, A61K 31/19

(54) **PARTICULES ENROBEES D'IBUPROFENE CRISTALLIN GRANULE**
UMHÜLLTE TEILCHEN BESTEHEND AUS KRISTALLISIERTEM GRANULIERTEM IBUPROFEN
PARTICLES COATED WITH GRANULATED CRYSTALLINE IBUPROFEN

(30) Priorité: 06.11.1998 FR 9814033
(43) Date de publication de la demande: 29.08.2001
(73) Titulaire: ETHYPHARM, 78550 Houdan (FR)
(72) Inventeur: ZUCCARELLI, Jean-Marc, F-06600 Antibes (FR); CHAUVEAU, Charles, André, F-06560 Valbonne (FR); DEMICHELIS, Gilles, F-06130 Grasse (FR); JEAN, Karine, F-06800 Cagnes sur Mer (FR)
(74) Mandataire: Touati, Catherine
(86) Numéro de dépôt international: PCT/FR1999/002682
(87) Numéro de publication internationale: WO 2000/027368

(56) Documents cités:
- WO-A-91/15194
- WO-A-93/01805
- GB-A- 2 178 313
- US-A- 4 588 612
- US-A- 4 835 187
- US-A- 5 084 278
- US-A- 5 191 114
- US-A- 5 215 755

## Description

L'invention a pour objet des particules enrobées à base d'ibuprofène cristallin granulé, de ses sels ou de ses esters pharmaceutiquement acceptables, qui comportent un enrobage, obtenu dans un appareil à lit fluidisé avec une dispersion hydroalcoolique, assurant le masquage du goût désagréable de l'ibuprofène ainsi que la réduction significative de son effet irritant au niveau de la gorge après déglutition et la libération substantiellement immédiate dudit ibuprofène dès que les particules atteignent le milieu gastrique.

Elle vise également le procédé de préparation desdites particules.

Les particules enrobées en question sont constituées par des microcristaux d'ibuprofène granulés.

Le brevet US 5,215,755 décrit des comprimés dans lesquels l'ibuprofène est présent sous la forme de granules comportant un enrobage à base d'hydroxyéthylcellulose ou d'un mélange hydroxyéthylcellulose/hydroxypropylméthylcellulose. Cet enrobage permet de réaliser un meilleur compromis entre masquage de goût et biodisponibilité, compromis que l'utilisation d'éthylcellulose seule ou en mélange avec d'autres polymères d'enrobage déjà connus ne permettait pas d'obtenir.

Le brevet US 5,814,332 décrit des particules d'ibuprofène encapsulées par coacervation du principe actif avec des polymères cellulosiques et de la gélatine.

Les brevets US 4,835,186 et US 4,835,187 décrivent des poudres d'ibuprofène obtenues par méthode de séchage par atomisation, plus connue sous le nom de nébulisation, de suspensions de silice colloïdale dans des solutions de solvants organiques d'ibuprofène et de matériau cellulosique tel que l'éthylcellulose, l'hydroxyéthylcellulose, l'hydroxypropylméthylcellulose, l'acéto-phtalate de cellulose

L'invention a pour but de fournir de nouvelles particules à base d'ibuprofène cristallin présentant une palatabilité neutre, sans goût et masquant l'effet irritant du principe actif. Leur répartition granulométrique et leurs caractéristiques physiques permettent leur utilisation en particulier dans la fabrication de comprimés multiparticulaires à délitement rapide dans la bouche sous l'action de la salive, conformément au brevet FR2679451 et conduisent à une libération substantiellement immédiate du principe actif.

La Société Demanderesse a eu le mérite de trouver, à l'issue de recherches approfondies, que ce but était atteint dès lors que l'on a recours à un procédé de granulation et d'enrobage dans un appareil à lit fluidisé avec un mélange essentiellement à base d'éthylcellulose, d'hydroxypropylméthylcellulose et de silice à propriétés antistatique et perméabilisante, dans des proportions définies.

Il s'ensuit que les particules conformes à l'invention à base de microcristaux granulés d'ibuprofène, de ses isomères et de ses sels pharmaceutiquement acceptables, sont caractérisées par le fait qu'elles comportent un enrobage constitué par un mélange comprenant:
A) de 5 à 50%, de préférence de 10 à 30% en poids par rapport à l'ibuprofène d'éthylcellulose,
B) de 10 à 60%, de préférence de 15 à 50% en poids par rapport à l'éthylcellulose, d'hydroxypropylméthylcellulose, et
C) de 0,1 à 40%, de préférence de 3 à 25% en poids par rapport à l'éthylcellulose, de silice à propriétés antistatique et perméabilisante,
l'enrobage ainsi constitué, dont au moins l'un des constituants peut être utilisé pour la granulation des microcristaux d'ibuprofène conduisant auxdites particules, assurant le masquage du goût désagréable de l'ibuprofène ainsi que la réduction significative de son effet irritant au niveau de la gorge après déglutition et la libération substantiellement immédiate de l'ibuprofène dès que les particules sont placées dans un milieu aqueux.

Le principe actif est constitué par l'ibuprofène cristallin ou l'un de ses sels ou esters pharmaceutiquement acceptables.

Le principe actif est disponible dans le commerce sous la forme de microcristaux dont la taille moyenne est comprise entre 20 et 80 µm.

L'inconvénient d'une telle granulométrie réside dans le fait que l'enrobage par les méthodes consistant à pulvériser une solution d'enrobage sur ces microcristaux dans un appareil à lit fluidisé est difficile et long.

Pour remédier à cet inconvénient, conformément à l'invention, les microcristaux d'ibuprofène sont granulés et enrobés de façon à conduire à des particules qui présentent une granulométrie telle qu'au moins 80% des particules ont une taille comprise entre 100 et 500 µm , et moins de 15% des particules ont une taille inférieure à 100 µm.

Dans les particules enrobées conformes à l'invention, l'ibuprofène conserve son intégrité physico-chimique, la granulation et l'enrobage ne modifiant en rien les propriétés intrinsèques du principe actif.

La silice à propriétés antistatique et perméabilisante (C), peut être choisie dans le groupe comprenant notamment la silice colloïdale, notamment celle commercialisée sous la marque AEROSIL®, et préférentiellement la silice précipitée, notamment celle commercialisée sous la marque SYLOID® FP244 et leurs mélanges.

De façon avantageuse, on peut utiliser en outre un agent (D) favorisant directement ou indirectement la solubilisation de l'ibuprofène, lequel agent est choisi dans le groupe comprenant notamment le mannitol, l'amidon, les bases auto-émulsionnables pharmaceutiquement acceptables, les polyvinylpyrrolidones, les macrogols glycérides stéariques plus connus sous le nom de gélucire, les sels alcalins d'origine organique,tels que le bicarbonate de sodium, les agents tensio-actifs tels que le lauryl sulfate de sodium et leurs mélanges. Cet agent (D) est présent dans des proportions pouvant aller jusqu'à 50% en poids, de préférence jusqu'à 35% en poids par rapport à l'ibuprofène.

Selon un mode de réalisation avantageux, les particules d'ibuprofène sont granulées avec au moins un agent favorisant la solubilisation de l'ibuprofène, choisi préférentiellement dans le groupe comprenant les macrogols glycérides stéariques, et l'hydroxypropylméthylcellulose, et sont enrobées à l'aide d'un mélange éthylcellulose/hydroxypropylméthylcellulose dans des proportions permettant de masquer le goût et l'effet irritant de l'ibuprofène et d'une silice à propriétés antistatique et perméabilisante, notamment de la silice précipitée, ce mode de réalisation conduisant à une biodisponibilité optimale de l'ibuprofène.

L'on obtient ainsi un masquage du goût et de l'effet irritant tout aussi satisfaisant qu'avec l'autre mode de préparation des particules, mais avec une rapidité de libération du principe actif dans les milieux aqueux optimisée.

Selon un autre mode de réalisation avantageux, les microcristaux d'ibuprofène sont granulés en présence de microcristaux d'un sel alcalin d'origine organique en tant qu'agent favorisant la solubilisation d'ibuprofène et d'une solution comprenant de l'hydroxypropylméthylcellulose et/ou de la polyvinylpyrrolidone. Ce sel alcalin est préférentiellement le bicarbonate de sodium, qui crée, en se dissolvant dans les liquides gastro-intestinaux, un micro-pH alcalin favorisant la solubilisation des particules d'ibuprofène.

Les particules ainsi constituées sont ensuite enrobées avec le mélange d'enrobage selon l'invention.

Selon un autre mode de réalisation avantageux, l'enrobage, toujours lorsqu'au moins l'un de ses constituants est utilisé pour la granulation des microcristaux d'ibuprofène, comporte un agent favorisant la solubilisation qui peut être un agent soluble tel que le mannitol ou un agent gonflant tel que l'amidon. Si l'on utilise un agent soluble, il cristallise à la surface des particules d'ibuprofène, et en milieu acide il se solubilise et laisse des pores qui permettent aux liquides physiologiques d'entrer à l'intérieur de la particule. Si l'on utilise un agent gonflant, un phénomène d'éclatement complémentaire de la particule se produit.

Les particules conformes à l'invention permettent une dissolution optimale de l'ibuprofène dans des milieux aqueux. La vitesse de dissolution des particules est telle que dans une solution tampon de pH 7,2, 80% de l'ibuprofène est libéré en 30 minutes et de préférence en 15 minutes.

L'invention a également pour objet un procédé de préparation de particules enrobées à base de microcristaux d'ibuprofène. Ce procédé comporte, simultanément ou successivement, une phase consistant à granuler les microcristaux d'ibuprofène et une phase consistant à les enrober à l'aide d'un enrobage constitué par un mélange
A) de 5 à 50%, de préférence de 10 à 30% en poids par rapport à l'ibuprofène, d'éthylcellulose,
B) de 10 à 60%, de préférence de 15 à 50% en poids par rapport à l'éthylcellulose, d'hydroxypropylméthylcellulose et
C) de 0,1 à 40%, de préférence de 3 à 25% en poids par rapport à l'éthylcellulose, de silice à propriétés antistatique et perméabilisante, au moins l'un des constituants du mélange servant à l'enrobage peut être utilisé pour la granulation des microcristaux d'ibuprofène.

Lors de la granulation et/ou de l'enrobage on peut utiliser en outre un agent (D) favorisant la solubilisation de l'ibuprofène qui est choisi dans le groupe comprenant le mannitol, l'amidon, les bases auto-émulsionnables pharmaceutiquement acceptables, les polyvinylpyrrolidones, les macrogols glycérides stéariques , les sels alcalins d'origine organique, les agents tensio-actifs et leurs mélanges. Cet agent (D) est alors présent dans des proportions pouvant aller jusqu'à 50% en poids, de préférence jusqu'à 35% en poids par rapport à l'ibuprofène.

Le procédé conforme à l'invention est mis en oeuvre dans un lit fluidisé dans des conditions de températures telles que la température de l'ibuprofène soit toujours maintenue à une température inférieure aux températures de fusion et de sublimation de l'ibuprofène. Selon un mode de réalisation particulier du procédé de l'invention la température de l'ibuprofène est toujours maintenue au dessous de 45°C, de préférence au dessous de 30°C.

En raison de l'utilisation d'un procédé de granulation et d'enrobage conduit en lit fluidisé, l'ibuprofène n'est pas mis en solution, il conserve ainsi son intégrité physico-chimique de façon optimale. Par ailleurs l'utilisation de basses températures permet d'éviter tout changement d'état et tout risque de dégradation du principe actif.

Selon un premier mode de réalisation, les phases de granulation et d'enrobage s'effectuent simultanément en mouillant les microcristaux d'ibuprofène avec une suspension hydroalcoolique comprenant notamment de l'éthylcellulose et de l'hydroxypropylméthylcellulose et une silice à propriétés antistatique et perméabilisante.

Selon un autre mode de réalisation du procédé selon l'invention, la phase de granulation est effectuée dans une première étape en utilisant au moins un agent favorisant la solubilisation, choisi dans le groupe comprenant notamment les macrogols glycérides stéariques et l'hydroxypropylméthylcellulose, la phase d'enrobage étant ensuite effectuée dans une deuxième étape en utilisant une silice à propriétés antistatique et perméabilisante et un mélange d'éthylcellulose, hydroxypropylméthylcellulose dans des proportions permettant de masquer le goût et l'effet irritant de l'ibuprofène et conduisant à une libération substantiellement immédiate de l'ibuprofène.

Dans un autre mode de réalisation avantageux du procédé selon l'invention dans la première étape de granulation, des microparticules d'ibuprofène sont mélangées à des microcristaux d'un sel alcalin d'origine organique en tant qu'agent favorisant la solubilisation de l'ibuprofène et le mélange ainsi obtenu est granulé avec une dispersion hydroalcoolique comprenant de l'hydroxypropylméthylcellulose et/ou de la polyvinylpyrrolidone. La phase d'enrobage est effectuée ensuite avec un mélange d'enrobage selon l'invention.

### EXEMPLES : Préparation de granulés enrobés d'ibuprofène

### Exemple 1 :

La formule unitaire du granulé enrobé est la suivante:

| | |
|---|---|
| Ibuprofène | 200,00 |
| Ethylcellulose N7 | 40,00 |
| Silice colloïdale | 3,00 |
| Hydroxypropylméthylcellulose | 8,00 |
| | 251,00 mg |

Ces granulés sont préparés selon le mode opératoire suivant.

Dans un premier temps, on prépare une dispersion d'enrobage. Pour cela on introduit 24 g d'hydroxypropylméthylcellulose dans 390 g d'eau purifiée et on agite jusqu'à dissolution complète de l'hydroxypropylméthylcellulose. On introduit par ailleurs 9 g de silice colloïdale et 120g d'éthylcellulose N7 dans 110 g d'alcool et on agite jusqu'à l'obtention d'une dispersion homogène.

On mélange alors la solution et la dispersion obtenues ci-dessus et on maintient l'agitation pour éviter toute sédimentation. On obtient ainsi une dispersion d'enrobage.

Dans un second temps, on prépare les granulés enrobés. Pour cela, on introduit 600 g d'ibuprofène dans la cuve d'un appareil à lit fluidisé de type GLATT GPCG1 et on fluidise l'ibuprofène dans des conditions telles que sa température soit maintenue entre 20 et 40°C. Puis, on pulvérise la dispersion d'enrobage précédente sur le lit d'ibuprofène obtenu ci-dessus de façon à ce que la température du produit reste maintenue entre 15 et 30°C.

On pulvérise 50% de la dispersion d'enrobage pendant environ 1 heure, puis on sèche pendant 2 à 5 minutes. Le granulé obtenu est calibré sur une grille de 400 µm d'ouverture de maille. Le granulé calibré est ensuite enrobé avec la dispersion d'enrobage restante pendant approximativement 1 h 30 minutes. Puis on sèche le granulé enrobé pendant 5 minutes environ.

Le granulé enrobé contient 79,7% en poids d'ibuprofène.

Sur les granulés ainsi obtenus, on réalise une cinétique de dissolution à l'aide d'un appareil type 4 décrit dans l'USP XXIII page 1794, dans un milieu tamponné à pH=7,2, avec un volume de dissolution de 900ml. Les résultats obtenus sont indiqués ci-après:

| temps de dissolution en mn | ibuprofène dissous en % |
|---|---|
| 15 | 88,1 |
| 30 | 100 |

### Exemple 2 :

La formule unitaire du granulé enrobé est la suivante :

| | |
|---|---|
| Ibuprofène | 200,00 mg |
| Ethylcellulose N7 | 40,00 mg |
| Hydroxypropylméthylcellulose (HPMC) | 8,00 mg |
| Silice précipitée (Syloïd® FP244) | 13,70 mg |
| | 261,70 mg |

Ces granulés sont préparés selon le mode opératoire suivant.

Dans un premier temps, on prépare une dispersion d'enrobage. Pour cela, on introduit 24 g d'HPMC dans 459g d'eau purifiée et on agite jusqu'à dissolution complète de l'HPMC. On introduit par ailleurs 41,9 g de silice précipitée commercialisée sou la dénomination Syloïd®FP244 et 120 g d'éthylcellulose N7 dans 1362g d'alcool et on agite jusqu'à l'obtention d'une dispersion homogène.

On mélange alors la solution et la dispersion obtenues ci-dessus et on maintient l'agitation pour éviter toute sédimentation. On obtient ainsi une dispersion d'enrobage.

Dans un second temps, on prépare les granulés enrobés. Pour cela on introduit 600g d'ibuprofène dans la cuve d'un appareil à lit fluidisé de type Glatt GPCG1 et on fluidise l'ibuprofène dans des conditions telles que sa température soit maintenue entre 20°C et 40°C. Puis on pulvérise la dispersion d'enrobage précédente sur le lit d'ibuprofène obtenu ci-dessus de façon à ce que la température du produit soit maintenue entre 15°C et 30°C.

On pulvérise 50% de la dispersion d'enrobage pendant environ 1 heure puis on sèche pendant 2 à 5 minutes. Le granulé obtenu est calibré sur une grille de 400 µm d'ouverture de maille. Le granulé calibré est ensuite enrobé avec la dispersion d'enrobage restante pendant approximativement une heure trente minutes. Puis on sèche le granulé enrobé pendant 5 minutes environ.

Le granulé enrobé obtenu contient 76,4% en poids d'ibuprofène.

### Exemple 3 :

La formule unitaire du granulé enrobé est la suivante :

| | |
|---|---|
| Ibuprofène | 200,00 mg |
| Bicarbonate de sodium | 81,70 mg |
| Ethylcellulose | 59,90 mg |
| HPMC | 28,60 mg |
| Silice colloïdale | 5,50 mg |
| | 375,70 mg |

### Préparation des granulés

Dans un premier temps, on prépare une dispersion de granulation en dissolvant 50g d'HPMC dans 600 ml d'eau purifiée, puis on ajoute 3 g de silice colloïdale et on maintient l'agitation pour éviter toute sédimentation. Dans un second temps, on prépare les granulés. Pour cela, on introduit 600g d'ibuprofène et 245g de bicarbonate de sodium dans la cuve d'un appareil à lit fluidisé de type Glatt GPCG1 et on fluidise le mélange de poudres dans des conditions telles que sa température soit maintenue entre 20°C et 40°C. Puis on pulvérise la dispersion de granulation précédente sur le lit de poudres obtenu ci-dessus de façon à ce que la température du produit soit maintenue entre 15°C et 30°C.

On pulvérise la dispersion de granulation pendant environ 1 heure 30 minutes puis on sèche pendant 2 à 5 minutes. Les granulés obtenus sont calibrés sur une grille de 500 µm d'ouverture de maille.

### Préparation des granulés enrobés.

Ces granulés sont préparés selon le mode opératoire suivant.

Dans un premier temps, on prépare une dispersion d'enrobage. Pour cela on introduit 24g d'HPMC dans 390g d'eau purifiée et on agite jusqu'à dissolution complète de l'HPMC. On introduit par ailleurs 9g de silice colloïdale et 120g d'éthylcellulose N7 dans 1160g d'alcool et on agite jusqu'à l'obtention d'une dispersion homogène.

On mélange alors la solution et la dispersion obtenues ci-dessus et on maintient l'agitation pour éviter toutes sédimentation. On obtient ainsi une dispersion d'enrobage.

Dans un second temps, on prépare les granulés enrobés. Pour cela, on introduit 600g de granulés d'ibuprofène préparés à l'étape précédente dans la cuve d'un appareil à lit fluidisé de type Glatt GPCG1 et on fluidise les granulés dans des conditions telles que leur température soit maintenue entre 20°C et 40°C.

Puis on pulvérise la dispersion d'enrobage précédente sur les granulés de façon à ce que la température du produit soit maintenue entre 15°C et 30°C.

On pulvérise 50% de la dispersion d'enrobage pendant environ 1 heure puis on sèche pendant 2 à 5 minutes. Les granulés obtenus sont calibrés sur une grille de 500 µm d'ouverture de maille. Les granulés calibrés sont ensuite enrobés avec la dispersion d'enrobage restante pendant approximativement une heure, puis on sèche les granulés enrobés pendant 5 minutes environ.

Les granulés enrobés obtenus contiennent 53,2% en poids d'ibuprofène.

### Exemple 4 :

La formule unitaire du granulé enrobé est la suivante :

| | |
|---|---|
| Ibuprofène | 200,00 mg |
| Amidon de maïs | 75,00 mg |
| Ethylcellulose | 50,00 mg |
| HPMC | 10,00 mg |
| Silice colloïdale | 3,50 mg |
| | 338,50 mg |

### Préparation des granulés :

Les granulés sont préparés selon le mode opératoire suivant:

Pour cela, on introduit 600g d'ibuprofène et 150g d'amidon de maïs dans un mélangeur granulateur à sacs de type Lödige et on granule avec 550g d'eau purifiée. Les granulés obtenus sont séchés à l'étuve puis calibrés sur une grille de 500 µm d'ouverture de maille.

### Préparation des granulés enrobés :

Ces granulés enrobés sont préparés selon le mode opératoire suivant :

Dans un premier temps, on prépare une dispersion d'enrobage contenant de l'HPMC et de l'amidon de maïs. Pour cela, on introduit 20g d'HPMC dans 333g d'eau purifiée, puis on agite jusqu'à dissolution complète de l'HPMC puis on ajoute 50g d'amidon de maïs et l'on agite jusqu'à l'obtention d'une dispersion homogène.

On introduit par ailleurs 3,5g de silice colloïdale et 100g d'éthylcellulose N7 dans 833g d'alcool et on agite jusqu'à l'obtention d'une dispersion homogène.

On mélange alors les deux dispersions obtenues ci-dessus et on maintient l'agitation pour éviter toute sédimentation. On obtient ainsi une dispersion d'enrobage.

Dans un second temps, on prépare les granulés enrobés. Pour cela, on introduit 500g de granulés d'ibuprofène préparés à l'étape précédente dans la cuve d'un appareil à lit fluidisé de type Glatt GPCG1 et on fluidise les granulés dans des conditions telles que leur température soit maintenue entre 20°C et 40°C.

Puis on pulvérise la dispersion d'enrobage précédente sur les granulés de façon à ce que la température du produit soit maintenue entre 15°C et 30°C.

On pulvérise 50% de la dispersion d'enrobage pendant environ 1 heure puis on sèche pendant 2 à 5 minutes. Les granulés obtenus sont calibrés sur une grille de 500 µm d'ouverture de maille. Les granulés calibrés sont ensuite enrobés avec la dispersion d'enrobage restante pendant approximativement une heure, puis on sèche les granulés enrobés pendant 5 minutes environ.

Les granulés enrobés obtenus contiennent 59,1% en poids d'ibuprofène.

## Revendications

1. Particules enrobées à base de microcristaux granulés d'ibuprofène, de ses isomères et de ses sels pharmaceutiquement acceptables, **caractérisées par le fait qu'**elles comportent un enrobage constitué par un mélange comprenant:
A) de 5 à 50%, de préférence de 10 à 30% en poids par rapport à l'ibuprofène d'éthylcellulose,
B) de 10 à 60%, de préférence de 15 à 50% en poids par rapport à l'éthylcellulose, d'hydroxypropylméthylcellulose, et
C) de 0,1 à 40%, de préférence de 3 à 25% en poids par rapport à l'éthylcellulose, de silice à propriétés antistatique et perméabilisante,
l'enrobage ainsi constitué, dont au moins l'un des constituants peut être utilisé pour la granulation des microcristaux d'ibuprofène conduisant auxdites particules, assurant le masquage du goût désagréable de l'ibuprofène ainsi que la réduction significative de son effet irritant au niveau de la gorge après déglutition et la libération substantiellement immédiate de l'ibuprofène dès que les particules sont placées dans un milieu aqueux.

2. Particules selon la revendication 1, **caractérisées par le fait que** la silice à propriétés antistatique et perméabilisante (C) est la silice précipitée.

3. Particules selon l'une des revendications 1 ou 2, **caractérisées par le fait qu'**elles comportent en outre un agent (D) favorisant la solubilisation de l'ibuprofène qui est choisi dans le groupe comprenant le mannitol, l'amidon, les bases auto-émulsionnables pharmaceutiquement acceptables, les polyvinylpyrrolidones, les macrogols glycérides stéariques , les sels alcalins d'origine organique, les agents tensio-actifs et leurs mélanges, cet agent (D) pouvant également être utilisé pour la granulation de l'ibuprofène cristallisé.

4. Particules selon l'une des revendications 1 à 3, **caractérisées par le fait que** la répartition granulométrique des particules est telle qu'au moins 80% des particules ont une taille comprise entre 100 et 500µm, et moins de 15% des particules ont une taille inférieure à 100µm.

5. Particules selon l'une des revendications 1 à 4, **caractérisées par le fait que** dans une solution tampon de pH 7,2, 80% de l'ibuprofène est libéré en 30 minutes et de préférence en 15 minutes.

6. Procédé de préparation de particules enrobées à base de microcristaux granulés d'ibuprofène, de ses isomères et de ses sels pharmaceutiquement acceptables, **caractérisé par le fait qu'**il comporte, simultanément ou successivement, les phases consistant à granuler les microcristaux d'ibuprofène et à les enrober à l'aide d'un mélange comprenant:
A) de 5 à 50%, de préférence de 10 à 30% en poids par rapport à l'ibuprofène d'éthylcellulose,
B) de 10 à 60%, de préférence de 15 à 50% en poids par rapport à l'éthylcellulose, d'hydroxypropylméthylcellulose, et
C) de 0,1 à 40%, de préférence de 3 à 25% en poids par rapport à l'éthylcellulose, de silice à propriétés antistatique et perméabilisante,
l'un au moins des constituants du mélange servant à l'enrobage peut être utilisé pour la granulation des microcristaux d'ibuprofène.

7. Procédé selon la revendication 6, **caractérisé par le fait que** les phases de granulation et d'enrobage s'effectuent simultanément.

8. Procédé selon la revendication 6 ou 7, **caractérisé par le fait que** le procédé est mis en oeuvre dans un appareil à lit fluidisé avec une dispersion hydroalcoolique, dans des conditions telles que la température de l'ibuprofène soit toujours inférieure à 45°C, de préférence inférieure à 30°C.

9. Procédé selon l'une des revendications 6 à 8, **caractérisé par le fait que** lors de la granulation et/ou de l'enrobage on utilise en outre un agent (D) favorisant la solubilisation de l'ibuprofène qui est choisi dans le groupe comprenant le mannitol, l'amidon, les bases auto-émulsionnables pharmaceutiquement acceptables, les polyvinylpyrrolidones, les macrogols glycérides stéariques , les sels alcalins d'origine organique, les agents tensio-actifs et leurs mélanges.

## Claims

1. Coated particles based on granulated microcrystals of ibuprofen, its isomers and its pharmaceutically acceptable salts, **characterized in that** they have a coating consisting of a mixture comprising
A) from 5 to 50% by weight, preferably from 10 to 30% by weight, of ethyl cellulose, based on the ibuprofen,
B) from 10 to 60% by weight, preferably from 15 to 50% by weight, of hydroxypropyl methyl cellulose, based on the ethyl cellulose, and
C) from 0.1 to 40% by weight, preferably from 3 to 25% by weight, of silica with antistatic and permeabilizing properties, based on the ethyl cellulose,
said coating - of which at least one of the constituents can be used for the granulation of the ibuprofen microcrystals to produce said particles - masking the unpleasant taste of the ibuprofen, significantly reducing its irritant effect on the throat after swallowing, and releasing the ibuprofen substantially immediately when the particles are placed in an aqueous medium.

2. Particles according to Claim 1, **characterized in that** the silica with antistatic and permeabilizing properties (C) is precipitated silica.

3. Particles according to one of Claims 1 or 2, **characterized in that** they also contain an agent (D) favouring the solubilization of the ibuprofen, said agent being selected from the group comprising mannitol, starch, pharmaceutically acceptable self-emulsifying bases, polyvinylpyrrolidones, stearic macrogol glycerides, alkali metal salts of organic origin, surfactants and mixtures thereof, it also being possible for said agent (D) to be used for the granulation of the crystalline ibuprofen.

4. Particles according to one of Claims 1 to 3, **characterized in that** the size distribution of the particles is such that at least 80% of the particles are between 100 and 500 µm and less than 15% of the particles are smaller than 100 µm.

5. Particles according to one of Claims 1 to 4, **characterized in that**, in a buffer solution of pH 7.2, 80% of the ibuprofen is released in 30 minutes and preferably in 15 minutes.

6. Process for the preparation of coated particles based on granulated microcrystals of ibuprofen, its isomers and its pharmaceutically acceptable salts, **characterized in that** it comprises, simultaneously or successively, phases consisting in granulating the ibuprofen microcrystals and coating them with a mixture comprising
A) 5 to 50% by weight, preferably 10 to 30% by weight, of ethyl cellulose, based on the ibuprofen,
B) 10 to 60% by weight, preferably 15 to 50% by weight, of hydroxypropyl methyl cellulose, based on the ethyl cellulose, and
C) 0.1 to 40% by weight, preferably 3 to 25% by weight, of silica with antistatic and permeabilizing properties, based on the ethyl cellulose;
at least one of the constituents of the mixture used for the coating can be used for the granulation of the ibuprofen microcrystals.

7. Process according to Claim 6, **characterized in that** the granulation and coating phases take place simultaneously.

8. Process according to Claim 6 or 7, **characterized in that** it is carried out in a fluidized bed apparatus with an aqueous-alcoholic dispersion under conditions such that the temperature of the ibuprofen is always below 45°C, preferably below 30°C.

9. Process according to one of Claims 6 to 8, **characterized in that**, in the granulation and/or coating phases, an agent (D) favouring the solubilization of the ibuprofen is also used, said agent being selected from the group comprising mannitol, starch, pharmaceutically acceptable self-emulsifying bases, polyvinylpyrrolidones, stearic macrogol glycerides, alkali metal salts of organic origin, surfactants and mixtures thereof

## Patentansprüche

1. Beschichtete Teilchen auf der Basis granulierter Mikrokristalle von Ibuprofen, seiner pharmazeutisch akzeptablen Isomere und Salze, **dadurch gekennzeichnet, daß** sie eine Beschichtung enthalten, gebildet durch eine Mischung, umfassend:
A) 5 bis 50 Gew.%, vorzugsweise 10 bis 30 Gew.%, Ethylcellulose in Bezug auf Ibuprofen,
B) 10 bis 60 Gew.%, vorzugsweise 15 bis 50 Gew.%, Hydroxypropylmethylcellulose in Bezug auf Ethylcellulose, und
C) 0,1 bis 40 Gew.%, vorzugsweise 3 bis 25 Gew.%, Siliciumdioxid in Bezug auf Ethylcellulose mit antistatischen und die Permeation verbessernden Eigenschaften,
wobei die so gebildete Beschichtung, von der mindestens einer der Bestandteile für die Granulierung der Mikrokristalle von Ibuprofen verwendet werden kann, die zu den Teilchen führt, die Maskierung des unangenehmen Geschmacks von Ibuprofen wie auch eine signifikante Reduktion des irritierenden Effekts in der Kehle nach dem Verschlucken und im wesentlichen die sofortige Freisetzung des Ibuprofens, sobald die Teilchen in einem wäßrigen Milieu plaziert sind, sicherstellt.

2. Teilchen gemäß Anspruch 1, **dadurch gekennzeichnet, daß** das Siliciumdioxid mit antistatischen und die Permeation verbessernden Eigenschaften (C) ausgefälltes Siliciumdioxid ist.

3. Teilchen gemäß einem der Ansprüche 1 oder 2, **dadurch gekennzeichnet, daß** sie außerdem ein Mittel (D) beinhalten, das die Solubilisierung des Ibuprofens begünstigt, das ausgewählt ist aus der Gruppe umfassend Mannit, Stärke, selbst emulgierbare pharmazeutisch akzeptable Basen, Polyvinylpyrrolidone, Stearinsäureglyceridmakrogole, alkalische Salze von organischem Ursprung, Tenside und ihre Mischungen, wobei das Mittel (D) außerdem für die Granulierung des kristallisierten Ibuprofens verwendet werden kann.

4. Teilchen gemäß einem der Ansprüche 1 bis 3, **dadurch gekennzeichnet, daß** die granulometrische Verteilung der Teilchen so aussieht, daß mindestens 80 % der Teilchen eine Größe zwischen 100 und 500 µm und weniger als 15 % der Teilchen eine Größe unterhalb von 100 µm aufweisen.

5. Teilchen gemäß einem der Ansprüche 1 bis 4, **dadurch gekennzeichnet, daß** in einer Pufferlösung mit einem pH von 7,2 80 % des Ibuprofens in 30 Minuten und vorzugsweise in 15 Minuten freigesetzt wird.

6. Verfahren zur Herstellung von beschichteten Teilchen auf Basis von mikrokristallinen Ibuprofenkörnern, seiner pharmazeutisch akzeptablen Isomere und Salze, **dadurch gekennzeichnet, daß** es gleichzeitig oder aufeinanderfolgend die Stufen enthält, bestehend aus Granulieren der mikrokristallinen Ibuprofenteilchen und ihre Beschichtung mit Hilfe einer Mischung, die folgendes umfaßt:
A) 5 bis 50 Gew.%, vorzugsweise 10 bis 30 Gew.%, Ethylcellulose in Bezug auf Ibuprofen,
B) 10 bis 60 Gew.%, vorzugsweise 15 bis 50 Gew.%, Hydroxypropylmethylcellulose in Bezug auf Ethylcellulose, und
C) 0,1 bis 40 Gew.%, vorzugsweise 3 bis 25 Gew.%, Siliciumdioxid in Bezug auf Ethylcellulose mit antistatischen und die Permeation verbessernden Eigenschaften,
wobei mindestens einer der Bestandteile der Mischung, die für die Beschichtung dient, für die Granulierung der Mikrokristalle des Ibuprofens verwendet werden kann.

7. Verfahren gemäß Anspruch 6, **dadurch gekennzeichnet, daß** die Stufen der Granulierung und der Beschichtung gleichzeitig durchgeführt werden.

8. Verfahren gemäß Anspruch 6 oder 7, **dadurch gekennzeichnet, daß** das Verfahren in einer Fließbettvorrichtung mit einer hydroalkoholischen Dispersion unter derartigen Bedingungen durchgeführt wird, daß die Temperatur des Ibuprofens immer unterhalb von 45°C, vorzugsweise unterhalb von 30°C, liegt.

9. Verfahren gemäß einem der Ansprüche 6 bis 8, **dadurch gekennzeichnet, daß** man während der Granulierung und/oder der Beschichtung weiterhin ein Mittel (D) verwendet, das die Solubilisierung des Ibuprofens begünstigt, ausgewählt aus der Gruppe umfassend Mannit, Stärke, selbst emulgierbare Basen, die pharmazeutisch akzeptabel sind, Polyvinylpyrrolidone, Stearinsäureglyceridmakrogole, alkalische Salze von organischem Ursprung, Tenside und ihre Mischungen.
